# EUROPÄISCHE PATENTSCHRIFT

(11) **EP 2 046 415 B1**
(45) Veröffentlichungstag und Bekanntmachung des Hinweises auf die Patenterteilung: **08.03.2017**
(21) Anmeldenummer: 07787989.8
(22) Anmeldetag: 27.07.2007
(51) Int. Cl.: A61M 1/10, A61M 1/12

(54) **KUNSTHERZ**
ARTIFICIAL HEART
COEUR ARTIFICIEL

(30) Priorität: 27.07.2006 DE 102006035548
(43) Veröffentlichungstag der Anmeldung: 15.04.2009
(73) Patentinhaber: Deutsches Zentrum für Luft- und Raumfahrt e.V., 51147 Köln (DE)
(72) Erfinder: VODERMAYER, Bernhard, 82205 Gilching (DE); SCHMID, Thomas, 82347 Bernried (DE)
(74) Vertreter: dompatent von Kreisler Selting Werner - Partnerschaft von Patent- und Rechtsanwälten mbB
(86) Internationale Anmeldenummer: PCT/EP2007/057774
(87) Internationale Veröffentlichungsnummer: WO 2008/012366

(56) Entgegenhaltungen:
- EP-A- 0 583 012
- WO-A-03/068292
- WO-A-2004/078234
- US-A- 4 922 910

## Beschreibung

Die Erfindung bezieht sich auf ein Kunstherz mit einer Blutpumpe mit einem Pumpenantrieb und einer Steuerung zum Steuern und Regeln des Pumpenantriebes.

Unter Kunstherzen sind vorliegend alle intra- und extrakorporalen Kunstherzen zu verstehen, die eine Blutpumpe aufweisen, also extrakorporale, voll- oder teilimplantierbare Kunstherzen, Herzunterstützungssysteme u.ä.. Bekannte Kunstherz-Implantate arbeiten mit einer festen Pumpfrequenz oder verfügen über eine einfache Sensorik, die den Motorstrom oder die Pumpkammer-Befüllung feststellt und steuert bzw. regelt. Extrakorporale Kunstherzen leiten EKG-Signale außen von der Patienten-Haut ab, die auf Grund der Entfernung zum Patienten-Herz recht schwach und ungenau sind. Intrakorporale Kunstherzen leiten die erforderlichen Pumpleistungen aus dem Motorstrom, dem Blutfluss, und ähnlichen Parametern ab. Diese Methoden sind nur begrenzt genau und unzuverlässig.

In DE 697 31 848 T2 wird ein Herzunterstützungssystem offenbart, das mit Hilfe von EKG-Elektroden gesteuert wird.

In DE 693 22 562 T2 wird eine Muskel-Stimulationsanordnung offenbart, bei der eine Elektrodensonde in der Nähe des Patienten-Herzens zur Ableitung von EKG- Signale verwendet wird.

US 4,922,910 beschreibt ein Kunstherz, das mit einem natürlichen Herzen verbunden ist. Es sind Elektroden vorgesehen, die die EKG-Signale des Herzens erfassen sollen. Eine elektrische Leitung, die die Elektroden mit dem Kunstherz verbindet, verläuft innerhalb der Wandung einer Blutleitung, die das Kunstherz mit dem natürlichen Herz verbindet. Die Elektrode ist an der Innenseite der Blutleitung ausgebildet.

Aufgabe der Erfindung ist es, ein Kunstherz zu schaffen, das einfach implantierbar ist und dauerhaft gute Elektroden-Signale zur Verfügung stellt. Diese Aufgabe wird erfindungsgemäß gelöst mit den Merkmalen des Patentanspruchs 1.

Gemäß der Erfindung ist ein mit der Steuerung verbundener elektrischer Sensor zur Detektion elektrischer Größen unmittelbar am Patientenherz vorgesehen. Die Steuerung steuert den Pumpenantrieb in Abhängigkeit von den durch den Sensor detektierten Signalen, Der Sensor kann aus einer oder mehreren EKG-Elektroden und einer oder mehreren Impedanz-Elektroden bestehen. Es werden also wichtige physiologische Parameter erfasst, die indirekt Auskunft darüber geben, welcher physiologische Bedarf an Blut-Volumenstrom vorliegt. Diese Information wird insbesondere über die unmittelbar am Patienten-Herzen platzierten bzw. platzierbaren EKG-Elektroden gewonnen, die myocardial evozierte Signale sehr detailgenau erfassen. Dabei wird davon ausgegangen, dass die kardial evozierten Signale noch vorhanden sind und den physiologischen Blut-Bedarf genau wiedergeben. Durch die unmittelbare Nähe des Ableitortes zum Patienten-Herz wird ein EKG-Signal gewonnen, das sehr detailliert ist und eine und eine genaue Steuerung und Regelung des Kunstherzens erlaubt. Mit Hilfe der durch den oder die Sensoren gewonnenen Informationen kann neben der genauen Steuerung der Blutpumpe bzw. des Pumpenantriebes auch eine ständige Überwachung des Patienten-Kreislaufes bzw. des Patienten-Herzens durchgeführt werden.

An einer Blutleitung des Kunstherz-Implantates sind eine Elektrode oder mehrere Elektroden vorgesehen. Die schlauchförmige Blutleitung weist an ihrer Außenseite eine Elektrode oder mehrere Elektroden auf. Die Elektroden sind relativ großflächig ausgebildet und als Ringe an der Außenseite der Blutleitung angeordnet.

Die Signal-Leitungen, die die Elektroden mit der Steuerung verbinden, sind in oder an dem Mantel der Blutleitung angeordnet. Hierdurch sind die Signal-Leitungen relativ gut geschützt gegen zu starke Bewegungen, zu starke Beugung oder Knicken.

Gemäß einer bevorzugten Ausgestaltung ist die Signal-Leitung bzw. sind die Signal-Leitungen schraubenartig an oder in dem Blutleitungs-Mantel verlegt. Hierdurch sind die minimalen Beugungsradien für die Signal-Leitungen sehr groß, so dass auch langfristig die Gefahr von Leitungsbrüchen gering ist.

Vorzugsweise ist ein elektrisches Modul zwischen der Elektrode und einem Steuerungscomputer vorgesehen, wobei mindestens eine Kurzschlussleitung das elektrische Modul umgeht und direkt mit dem Steuerungscomputer verbunden ist. Ein elektrisches Modul kann ein Verstärker, und/oder ein Filter, und/oder ein Analog/Digital-Wandler sein. Bei korrektem Betrieb des Moduls bzw. der in Reihe hintereinander angeordneten Module durchläuft das Elektroden-Signal alle elektrischen Module nacheinander, um anschließend verstärkt, gefiltert und digitalisiert in den Steuerungscomputer geleitet zu werden. Uber die Kurzschlussleitung bzw. die Kurzschlussleitungen kann das selbe Elektroden-Signal unter Umgehung eines, mehrerer oder aller elektrischer Module direkt an den Steuerungscomputer gesendet werden. Der Steuerungscomputer kann das Signal der Kurzschlussleitung ständig vergleichen mit dem von dem letzten elektrischen Modul kommenden Signal. Hierdurch wird eine Redundanz erzielt, die für die unerlässliche Betriebssicherheit eins lebenswichtigen Implantates, wie es das Kunstherz ist, sicherstellt.

Im folgenden werden unter Bezugnahme auf die Zeichnungen mehrere Ausführungsbeispiele der Erfindung näher erläutert.

Es zeigen:
- Fig. 1: eine schematische Darstellung eines Patienten-Herzens mit einem erfindungsgemäßen intrakorporalen Kunstherz-Implantat,
- Fig. 2: eine schematische Darstellung einiger Module der Kunstherz-Steuerung des Kunstherz-Implantates der Fig. 1,
- Fig. 3: eine alternative Ausbildung eine Anordnung von Elektroden an einer Sonde zur Detektion elektrischer Größen am Patienten-Herz, und
- Fig. 4: eine weitere Ausgestaltung von Elektroden zur Detektion elektrischer Größen am Patienten-Herz.

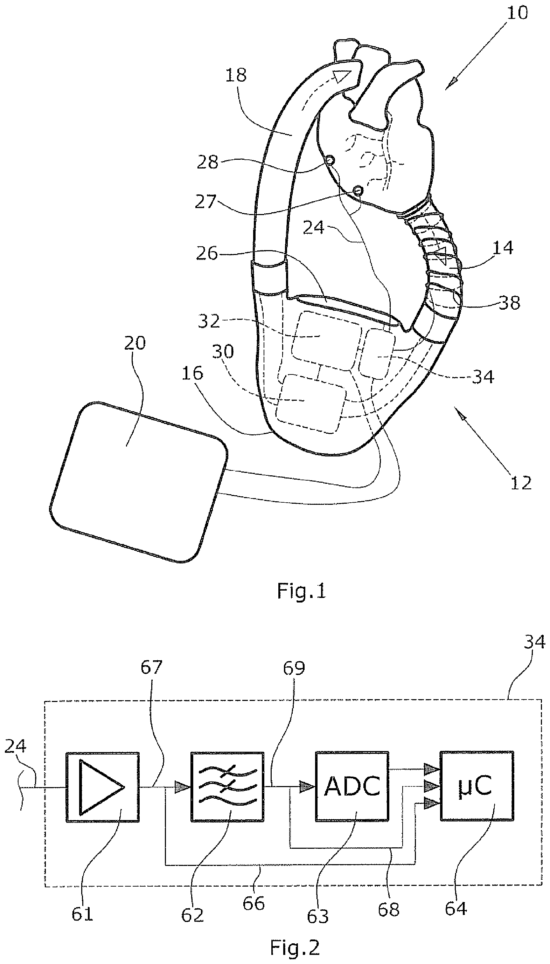

In der Fig. 1 ist ein Patienten-Herz 10 dargestellt, das durch ein Kunstherz-Implantat 12 unterstützt wird. Das Kunstherz-Implantat 12 ist ein sogenanntes Vollimplantat, weist also keine direkte physische Verbindung zur Exkorporalen auf.

Das Kunstherz-Implantat 12 weist eine Einlassleitung 14, die mit ihrer Einlassseite an dem linken Ventrikel des Patienten-Herzens 10 vernäht ist, eine Pumpeneinheit 16, in die die Einlassleitung 14 mündet, eine Auslassleitung 18, in die durch die Pumpeneinheit 16 Blut gepumpt wird und die in die Aorta des Patienten-Herzens 10 mündet, und eine intrakorporale Energieversorgung 20 auf, die über Signal- und Datenleitungen mit der Pumpeneinheit 16 elektrisch verbunden ist. Die Einlass- und die Auslassleitung 14, 18 sind Blutleitungen.

Ferner sind an der Pumpeneinheit 16 angeordnet sowie über entsprechende Signal-Leitungen 24 mit der Pumpeneinheit 16 verbundene Elektroden 26, 27, 28 vorgesehen, die EKG-Elektroden sind und der Ableitung myocardial evozierter Signale dienen.

Die Pumpeneinheit 16 weist eine das Patienten-Herz 10 unterstützende mechanische Blutpumpe 30 auf, die durch einen elektrischen Pumpenantrieb 32 angetrieben wird. Ferner weist die Pumpeneinheit 16 eine Steuerung 34 auf, die über Signal- bzw. Daten-Leitungen mit dem Pumpenantrieb 32, den Elektroden 26, 27, 28 sowie der Energieversorgung 20 verbunden ist. Die Steuerung 34 steuert den Pumpenantrieb 32 u. a. in Abhängigkeit von den durch die Elektroden 26, 27, 28 detektierten EKG-Signalen, beispielsweise synchron zu dem EKG-Signal. Die am Patienten-Herzen 10 angebrachten EKG-Elektroden 26, 27 sind an dem Patienten-Herzen 10 vernäht.

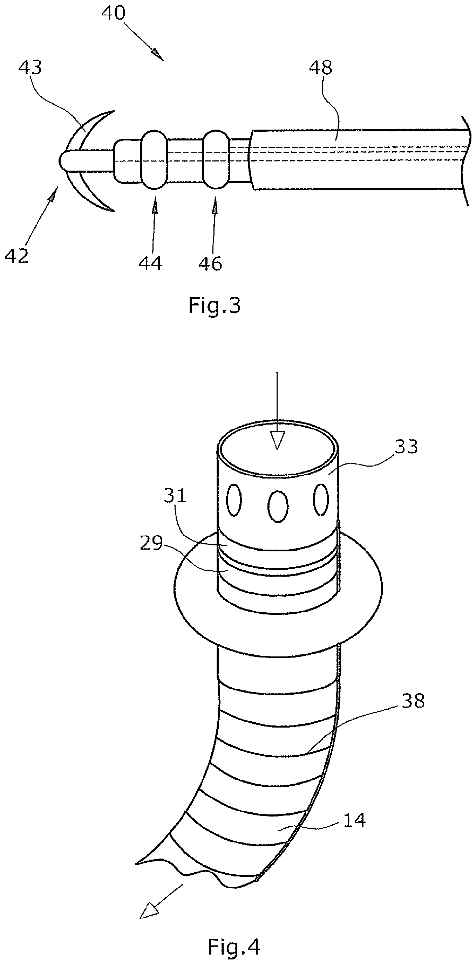

An dem offenen herzseitigen Ende der Einlassleitung 14 sind weitere Elektroden 29, 31 vorgesehen, die in Fig. 4 erkennbar sind. Die RingElektroden 29, 31 bestehen aus Metall oder einem elektrisch leitfähigem Kunststoff. Die als Ringelektroden 29, 31 sind über auf der schlauchförmigen Wand der Einlassleitung 14 verlegte schraubenartige Signal-Leitungen 38 mit der Steuerung 34 verbunden.

Die Signal-Leitung kann auch in die Einlassleitungs-Wand als Draht oder Gewebe in diese eingearbeitet sein. Die Signal-Leitung ist isoliert und damit knick- und bruchsicher in der Einlassleitungs-Wand untergebracht. Wenn für die Einlassleitung ein glattes Grundmaterial verwendet wird, beispielsweise ein Silikon oder PUR, kann die Signal-Leitung in die Einlassleitungs-Wand eingegossen werden. Wenn die Signal-Leitung 38 stärker dimensioniert oder ummantelt ist, kann sie die Einlassleitungs-Wand stützen und stabilisieren und auf diese Weise die Einlassleitungs-Wand vor Kollabieren schützen. Anstatt der schraubenförmig verlegten Signal-Leitung 38 kann diese alternativ auch beispielsweise im Inneren der Einlassleitung axial oder annähernd axial verlegt sein.

Um eine gute Verwachsung des distalen Einlassleitungs-Endes mit dem HerzGewebe sicher zu stellen, ist ein Endabschnitt der Einlassleitung 14 außenseitig mit einer Velouroberfläche 33 überzogen.

Die in der Fig. 1 dargestellten separaten EKG-Elektroden 27, 28 sind als sogenannte Fraktal-Elektroden ausgebildet, d. h. sie weisen an ihrem Ende eine selbstähnliche Fraktalstruktur auf, z. B. Romanesco. Hierdurch wird eine gute Signalqualität erreicht. Die Längen der Elektroden-Leitungen 24 sind fest vorgegeben. Es ist nicht vorgesehen, dass die Leitungen 24 während der Operation gekürzt werden können. An den steuerungsseitigen Ende der Signal-Leitungen 24, 38 sind Kontaktflächen angebracht, die in einer entsprechenden Klemme mit einer Klemmschraube fixiert und auf diese Weise an und mit der Steuerung 34 verbunden sind.

Je nach dem gewünschten Signaltyp, EKG-Signal oder Impedanz-Signal, werden die Elektroden 27, 28 auf dem linken Ventrikel in optimaler Position zur Potentiallinie positioniert, um ein optimal auswertbares EKG-Signal zu erhalten. Soll auch ein Vorhof-EKG zur Feststellung von Vorhofflimmern abgenommen werden, so ist auch dort eine Elektrode zu platzieren.

Zur Messung impedanz-cardiographischer Eigenschaften des Patienten-Herzens 10 müssen drei bis vier Elektroden vorgesehen sein, nämlich eine bis zwei Massen-Elektroden 26, eine Signal-Elektrode, die ein Signal von beispielsweise 150 kHz generiert, sowie eine Impedanz-Elektrode. Eine einzige Massen-Elektrode ist ausreichend, wenn ein großflächiges Gehäuse als Massen-Elektrode genutzt wird.

In der Fig. 3 ist eine alternativ zu der in Fig. 1 dargestellten Anordnung oder ergänzend hierzu einsetzbare Elektrodensonde 40 dargestellt, die an ihrem distalen Ende drei Elektroden 42, 44, 46 aufweist. Die End-Elektrode 42 weist einen Widerhaken 43 auf, der in das Myocard eingeführt und dort beispielsweise vernäht wird. Die beiden übrigen Elektroden 44, 46 sind ringförmig ausgebildet. Über den Abstand der drei Elektroden 42, 44, 46 zueinander ist die maximal Potentialgröße definiert. Eine der Elektroden 46 kann beispielsweise die Masse-Elektrode, eine andere Elektrode 44, 42, eine EKG-Elektrode bilden.

Die drei Elektroden 42 ,44, 46 sind über entsprechende isolierte Leitungen in einem Sondenmantel 48 knicksicher verlegt. Auf diese Weise werden langfristig Brüche der Signal-Leitungen vermieden.

In der Fig. 2 sind einige elektronische Module 61, 62, 63, 64 der Steuerung 34 dargestellt, nämlich ein Verstärker-Modul 61, ein Filter-Modul 62 und ein Analog/Digital-Wandler-Modul 63 sowie eine Steuerungscomputer 64. Es sind ferner zwei Kurzschlussleitungen 66, 68 vorgesehen, die die Signalleitung 67, 69 zwischen dem Verstärker-Modul 61 und dem Filter-Modul 62 bzw. zwischen dem Filter-Modul 62 und dem Analog/Digital-Wandler-Modul 63 direkt mit dem Steuerungscomputer 64 verbinden. Die steuerungscomputerseitigen Eingänge für die beiden Kurzschlussleitungen 66, 68 sind als Analog-Digital- Wandler ausgebildet.

Die analogen Signale der Elektroden werden über die Signalleitungen 24 an den Eingang des Verstärkermoduls 61 übertragen. Von dort aus wird das verstärkte Signal über die Signalleitung 67 an das Filter-Modul 62 gesendet. Das durch das Filter-Modul 62 gefilterte Elektroden-Signal wird über die Signalleitung 69 an das feinauflösende Analog/Digital-Wandler-Modul gesendet. Von dort aus wird das digitalisierte Elektroden-Signal an einen digitalen Eingang des Steuerungscomputers 64 geschickt.

Die Verbindung des bzw. der Analog/Digital-Wandler-Module 63 mit dem Steuerungscomputer 64 kann über einen I²C- oder SPI-Bus erfolgen.

Durch die Kurz schlussleitungen 66, 68 kann in dem Steuerungscomputer 64 einerseits das von dem Analog/Digital-Wandler-Modul 63 empfangene aufbereitete Elektroden-Signal auf weitgehende Übereinstimmung überprüft werden. Ferner kann bei Ausfall des Filter-Moduls 62 und/oder des Analog/Digital-Wandler-Moduls 63 das von dem Verstärker-Modul 61 kommende Sensor-Signal noch genutzt und weiter ausgewertet werden, wenn auch in verschlechterter Qualität.

Auf diese Weise wird mit relativ einfachen Mitteln eine hohe Kontrollierbarkeit und Redundanz realisiert. Ferner können auf diese Weise Module 62, 63 nachträglich kalibriert werden.

Die Steuerung 34 steuert die Leistung des Pumpenantriebes 32 in Abhängigkeit von den durch die Elektroden gemessenen EKG- und Impedanz-Signalen, beispielsweise schlag-synchron.

Die Elektroden (26, 27, 28, 29, 31), die mit der Blutbahn in Kontakt kommen, können mit gerinnungshemmenden Beschichtungen versehen sein, oder aber spezielle Oberflächen aufweisen, die keine oder nur eine kontrollierte Anlagerung von Blutblättchen zulassen. Dies gilt auch für die Elektroden, die außerhalb der Blutbahn in bestimmten Bereichen mit Blut in Berührung kommen können, in denen eine Thrombozyten-Anlagerung gefährlich sein kann.

## Patentansprüche

1. Kunstherz (12) mit
einer Blut-Einlassleitung (14) und/oder einer Blut-Auslassleitung (18), einer Blutpumpe (30) mit einem Pumpenantrieb (32), und
einer Steuerung (34) zum Steuern und Regeln des Pumpenantriebes (32), und
einer mit der Steuerung (34) verbundenen Elektrode (29, 31) zur Detektion elektrischer Größen am Patienten-Herz (10),
wobei die Steuerung (34) den Pumpenantrieb (32) in Abhängigkeit von den durch die Elektrode (29, 31) detektierten Signalen steuert,
wobei die Elektrode (29, 31) an der Blut-Einlassleitung (14) bzw. der Blut-Auslassleitung (18) vorgesehen ist, und
eine elektrische Signal-Leitung (38) zwischen der Elektrode (29, 31) und der Steuerung (34) in oder an der Wand der Blut-Einlassleitung (14) bzw. der Blut-Auslassleitung (18) angeordnet ist,
**dadurch gekennzeichnet, dass**
die Elektroden (29, 31) ringförmig an der Außenseite der Blut-Einlassleitung (14) und/oder der Blut-Auslassleitung (18) vorgesehen sind und unmittelbar am Patienten-Herzen (10) platziert sind,
wobei die Elektrode (29, 31) eine EKG-Elektrode ist.

2. Kunstherz (12) nach Anspruch 1, **dadurch gekennzeichnet, dass** die Signal-Leitung (38) schraubenartig an oder in der Wand der Einlassleitung (14) oder der Auslassleitung (18) verlegt ist.

3. Kunstherz (12) nach einem der Ansprüche 1 bis 2, **dadurch gekennzeichnet, dass** die Steuerung (34) ein elektrisches Modul (61, 62, 63) zwischen der Elektrode (27, 28) und einem Steuerungscomputer (64) aufweist, wobei mindestens eine Kurzschlussleitung (66, 68) das Modul (62, 63) umgeht und direkt mit dem Steuerungscomputer (64) verbunden ist.

## Claims

1. An artificial heart (12) comprising:
a blood inlet line (14) and/or a blood outlet line (18)
a blood pump (30) with a pump drive (32), and
a control (34) for controlling and regulating the pump drive (32), and
an electrode (29, 31) connected with the control (34) for detecting electrical values at a patient's heart (10),
the control (34) controlling the pump drive (32) as a function of the signals detected by the electrode (29, 31),
wherein the electrode (29, 31) is provided at the blood inlet line (14) or the blood outlet line (18), and
an electrical signal line (38) is provided between the electrode (29, 31) and the control (34) in or at the wall of the blood inlet line (14) or the blood outlet line (18),
**characterized in that**
the electrodes (29, 31) are provided in an annular shape on the outer side of the blood inlet line (14) and/or the blood outlet line (18) and are positioned immediately at a patient's heart (10),
wherein the electrode (29, 31) is an ECG electrode.

2. The artificial heart (12) of claim 1, **characterized in that** the signal line (38) is placed helically at or in the wall of the inlet line (14) or the outlet line (18).

3. The artificial heart (12) of one of claims 1 to 2, **characterized in that** the control (34) comprises an electric module (61, 62, 63) between the electrode (27, 28) and a control computer (64), wherein at least one short-circuit line (66, 68) bypasses the module (62, 63) and is directly connected with the control computer (64).

## Revendications

1. Coeur artificiel (12) avec
une tubulure d'entrée de sang (14) et/ou une tubulure de sortie de sang (18),
une pompe à sang (30) avec un entraînement de pompe (32) et
une commande (34) pour commander et réguler l'entraînement de pompe (32) et
une électrode (29, 31) connectée à la commande (34), pour la détection de grandeurs électriques au coeur du patient (10),
la commande (34) commandant l'entraînement de pompe (32) en fonction des signaux détectés par l'électrode (29, 31),
l'électrode (29, 31) étant prévue à la tubulure d'entrée de sang (14) ou à la tubulure de sortie de sang (18), et
une ligne électrique pour les signaux (38) entre l'électrode (29, 31) et la commande (34) étant disposée dans ou sur la paroi de la tubulure d'entrée de sang (14) ou de la tubulure de sortie de sang (18), **caractérisé en ce que**
les électrodes (29, 31) sont prévues à la façon d'un anneau sur la face extérieure de la tubulure d'entrée de sang (14) et/ou de la tubulure de sortie de sang (18) et sont placées directement auprès du coeur du patient (10),
l'électrode (29, 31) étant une électrode électrocardiographique.

2. Coeur artificiel (12) selon la revendication 1, **caractérisé en ce que** la ligne électrique pour les signaux (38) est placée à la façon d'une vis sur ou dans la paroi de la tubulure d'entrée (14) ou de la tubulure de sortie (18).

3. Coeur artificiel (12) selon l'une des revendications 1 à 2, **caractérisé**
**en ce que** la commande (34) comprend un module électrique (61, 62, 63) entre l'électrode (27, 28) et un ordinateur de commande (64), au moins une ligne de court-circuit (66, 68) contournant le module (62, 63) et étant directement connectée à l'ordinateur de commande (64).
